# EUROPEAN PATENT SPECIFICATION

(11) **EP 3 869 199 B1**
(45) Date of publication and mention of the grant of the patent: **09.02.2022**
(21) Application number: 20215445.6
(22) Date of filing: 20.02.2020
(51) Int. Cl.: G01N 33/569, C07K 14/005, C07K 14/165

(54) **A METHOD AND REAGENTS FOR THE DIAGNOSIS OF SARS-COV-2**
VERFAHREN UND REAGENZIEN ZUR DIAGNOSE VON SARS-COV-2
PROCÉDÉ ET RÉACTIFS POUR LE DIAGNOSTIC DU SARS-COV-2

(43) Date of publication of application: 25.08.2021
(62) Divisional of application: 20158626.0
(73) Proprietor: EUROIMMUN Medizinische Labordiagnostika AG, 23560 Lübeck (DE); Charité - Universitätsmedizin Berlin, 10117 Berlin (DE)
(72) Inventor: STEINHAGEN, Katja, 23627 Groß Grönau (DE); MESSING, Claudia, 23628 Klempau (DE); LATTWEIN, Erik, 23564 Lübeck (DE); STIBA, Konstanze, 18109 Rostock (DE); LINDHORST, Fabian, 23552 Lübeck (DE); NEUGEBAUER, Eva, 02763 Zittau (DE); MÜLLER, Marcel, 10117 Berlin (DE); CORMAN, Victor, 10117 Berlin (DE)

(56) References cited:
- WO-A2-2005/118813
- US-A1- 2005 112 559
- US-A1- 2006 188 519
- JIANG SHIBO ET AL: "An emerging coronavirus causing pneumonia outbreak in Wuhan, China: calling for developing therapeutic and prophylactic strategies", EMERGING MICROBES & INFECTIONS, vol. 9, no. 1, 1 January 2020 (2020-01-01) , pages 275-277, XP055785642, DOI: 10.1080/22221751.2020.1723441 Retrieved from the Internet: URL:https://www.ncbi.nlm.nih.gov/pmc/artic les/PMC7033706/pdf/TEMI_9_1723441.pdf>
- MEYER BENJAMIN ET AL: "Serological assays for emerging coronaviruses: Challenges and pitfalls", VIRUS RESEARCH, AMSTERDAM, NL, vol. 194, 23 March 2014 (2014-03-23), pages 175-183, XP029105622, ISSN: 0168-1702, DOI: 10.1016/J.VIRUSRES.2014.03.018

## Description

The present invention relates to a method for aiding in the diagnosis of a SARS-CoV-2 infection comprising the step of detecting the presence or absence of an IgA class antibody to SEQ ID NO1 in a blood sample from a subject and a use of an IgA class antibody to SEQ ID NO1 for aiding in the diagnosis of a SARS-CoV-2 infection , wherein the use is for the early diagnosis of a SARS-CoV-2 infection.

At the end of 2019, a rising number of pneumonia patients with unknown pathogen emerged from Wuhan, the capital of Hubei province, China, to nearly the entirety of China. A novel coronavirus was isolated and based on its phylogeny, taxonomy and established practice, the Coronavirus Study Group (CSG) recognized it as a sister to severe acute respiratory syndrome coronaviruses (SARS-CoVs) and labeled it as severe acute respiratory syndrome coronavirus 2 (SARS-CoV-2).

Although SARS-CoV-2 is generally less pathogenic than SARS-CoV and Middle East respiratory syndrome coronavirus (MERS-CoV), it has a relatively high transmissibility. Since symptoms may be mild and may be confused with a cold, there is the danger that patients are unaware that they have been infected and may help the virus spread further.

Corman *et al.* published a real time RT-PCR based assay for the detection of SARS-CoV-2 (Corman et al. (2020) Diagnostic detection of 2019-nCoV by real-time RT-PCR, https://www.who.int/docs/default-source/coronaviruse/protocol-v2-1.pdf?sfvrsn=a9ef618c 2). WO14045254 discloses assays for diagnosing a MERS infection.

US2005/0112559 discloses reagents derived from SARS-CoV nucleocapsid protein for the diagnosis of SARS-CoV.

WO2005118813 discloses immunogenic compositions associated with the SARS-CoV spike protein.

US2006/0188519 discloses immunoreactive peptides derived from SARS-CoV.

Jiang *et al.* (Jiang, S., Du, L., Shi, Z. Emerging Microbes and Infections, 2020 Vol. 9, 275-277) discuss therapeutic strategies related to SARS-CoV-2.

Meyer et al. (Virus research 194 (2014), 175-183) discuss serological assays relating to emerging corona viruses.

Hsueh *et al.,* reported that IgG could be detected as early as four days after the onset of a SARS infection, simultaneously or one day earlier than IgM and IgA (Hsue, P. R., Huang, L. M., Chen, P. J., Kao, C. L., and Yang P. C. (2004) Chronological evolution of IgM, IgA, IgG and neutralization antibodies after infection with SARS-associated coronavirus, Clinical Microbiology and Infection, 10(12), 1062-1066.

However, PCR-based assays have several shortcomings. In particular, a sample from the upper respiratory tract of the patient is required. Improper recovery of such a sample may lead to false-negative results. Moreover, the results may not reflect the disease status of the patient. Therefore, a serological assay would be desirable.

Therefore, a problem underlying the present invention is to provide an assay and reagents for the early serological detection of SARS-CoV-2.

The problems are solved by the subject matter of the independent and dependent claims.

In a first aspect, the problem is solved by a method for aiding in the diagnosis of a SARS-CoV-2 infection comprising the step of detecting the presence or absence of an IgA class antibody to SEQ ID NO1 in a blood sample from a subject.

In a preferred embodiment, the presence of an IgG and/or IgM class antibody to SEQ ID

NO1 is detected in addition to an IgA class antibody to SEQ ID NO1.

In a preferred embodiment, the sample is selected from the group comprising whole blood, serum or plasma.

In a preferred embodiment, the antibody is detected using a labeled secondary antibody, preferably binding to IgA class antibodies.

In a preferred embodiment, the IgA class antibody is detected using a method selected from the group comprising colorimetry, immunofluorescence, detection of enzymatic activity, chemiluminscence and radioactivity.

In a preferred embodiment, the infection is detected at an early stage.

In a 3^{rd} aspect, the problem is solved by a use of an IgA class antibody to SEQ ID NO1, for aiding in the diagnosis of a SARS-CoV-2 infection, wherein the use is for the early diagnosis of a SARS-CoV-2 infection.

The present invention is based on the inventors' surprising finding that antibodies against SEQ ID NO1 can be detected at an early stage of the infection, with IgA class antibodies becoming detectable earlier than IgG antibodies.

The term "diagnosis", as used herein, refers to any kind of procedure aiming to obtain information instrumental in the assessment whether a patient suffers or is likely or more likely than the average or a comparative subject, the latter preferably having similar symptoms, to suffer from certain a disease or disorder in the past, at the time of the diagnosis or in the future, to find out how the disease is progressing or is likely to progress in the future or to evaluate the responsiveness of a patient with regard to a treatment. In other words, the term "diagnosis" comprises not only diagnosing, but also prognosticating and/or monitoring the course of a disease or disorder. The subject is likely or more likely to suffer from a SARS-CoV-2 infection if an IgA and/or IgG and/or IgM antibody to SEQ ID NO1 is detected in samples from them.

The sample is preferably a mammalian, more preferably a human sample.

Therefore, the term "diagnosis" does preferably not imply that the diagnostic methods or agents according to the present invention will be definitive and sufficient to finalize the diagnosis on the basis of a single test, let alone parameter, but may refer to a contribution to what is referred to as a "differential diagnosis", *i.e.* a systematic diagnostic procedure considering the likelihood of a range of possible conditions on the basis of a range of diagnostic parameters. In a preferred embodiment, the term "SARS-CoV-2", as used herein, refers to a virus characterized by the genome deposited on GenBank under accession code MN908947 and derivatives thereof having at least 80, preferably 85, preferably 88, preferably 90, preferably 91, preferably 92, preferably 93, preferably 94, preferably 95, preferably 96, preferably 97, preferably 98, preferably 99, preferably 99.5, preferably 99.8, preferably 99.9 or 99.99 percent sequence identity over the entire genome nucleotide sequence. All data base entries used herein correspond to the version online at the filing date of the application. In a preferred embodiment, the term "diagnosis" means that the method or product or use may be used for aiding in the diagnosis of a disease or identifying a subject a risk of suffering from a disease. The term "diagnosis" may also refer to a method or agent used to choose the most promising treatment regime for a patient. In other words, the method or agent may relate to selecting a treatment regimen for a subject.

In a preferred embodiment, the method according to the present invention comprises the step providing the diagnostically useful carrier and a sample from a patient suspected of being infected, preferably a mammalian, more preferably a human patient. The carrier is coated with the polypeptide comprising SEQ ID NO1 or variant thereof. The carrier may then be contacted with the sample under conditions allowing for binding of any antibodies to the polypeptide comprising SEQ ID NO1 or variant thereof. The sample may then be removed and the carrier with the cell may be washed to remove any remaining sample. A secondary antibody or similar reagent or means binding to the antibody and carrying a detectable label may then be contacted with the carrier under conditions allowing formation of a complex between any bound antibody and the secondary antibody. The carrier may be washed then to remove non-bound secondary antibody. Finally, the presence of the antibody is detected by checking whether the secondary antibody may be detected.

In a preferred embodiment the method is used more than once to examine samples from the same patient, preferably on different days. For example, the presence or absence of antibodies may be detected on a daily basis over one or two weeks. In a preferred embodiment, least 2, 3, 4, 5, 6, 7, 8, 9 or 10 samples are examined on different days.

The method according to the present invention may also be used for screening the potency and the usefulness of an antiviral drug.

The method according to the present invention may also be used for screening whether donated blood is contaminated with coronavirus.

The antibody to be detected binds preferably specifically to SEQ ID NO1. Specific binding preferably means that the binding reaction is stronger than a binding reaction characterized by a dissociation constant of 1 × 10⁻⁵ M, more preferably 1 × 10⁻⁷ M, more preferably 1 × 10⁻⁸ M, more preferably 1 × 10⁻⁹ M, more preferably 1 × 10⁻¹⁰ M, more preferably 1 × 10⁻¹¹ M, more preferably 1 × 10⁻¹² M, as determined by surface plasmon resonance using Biacore equipment at 25 °C in PBS buffer at pH 7.

The teachings of the present invention may not only be carried out using polypeptides having the exact sequences referred to in this application explicitly, for example by function, name, sequence or accession number, or implicitly, but also using variants of such polypeptides.

The term "variant", as used herein, may refer to at least one fragment of the full length sequence referred to or a polypeptide comprising said fragment, more specifically to one or more amino acid or nucleic acid sequences which are, relative to the full-length sequence, truncated at one or both termini by one or more amino acids. Such a fragment comprises or encodes for a peptide having at least 10, 15, 25, 50, 75, 100, 150, 200, 250, 300, 400, 500 or 600 successive amino acids of the original sequence or for a variant thereof. For example, SEQ ID NO12 is an exemplary fragment that may be used.

The term "variant" relates not only to at least one fragment, but also a polypeptide or a fragment thereof comprising amino acid sequences, preferably a fragment comprising at least 25, more preferably 50, more preferably 200 successive amino acids, that are at least 40, 50, 60, 70, 75, 80, 85, 90, 92, 94, 95, 96, 97, 98 or 99 % identical to the reference amino acid sequence referred to or the fragment thereof, wherein amino acids other than those essential for the biological activity, for example the ability to bind specifically to an antibody of interest, or the fold or structure of the polypeptide are deleted or substituted and/or one or more such essential amino acids are replaced in a conservative manner and/or amino acids are added or deleted such that the biological activity of the polypeptide is at least partially preserved. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007): Clustal W and Clustal X version 2.0. Bioinformatics, 23, 2947-2948) is used applying default settings.

SARS-CoV-2-related publications on specific amino acid sequences such as Beal et al. may aid the skilled one in designing variants (Beal, J., Mitechell, T., Wyschogrod, W., Manthey, J, and Clore, A. (2020) Highly Distinguished Amino Acid Sequences of 2019-nCoV (Wuhan Coronavirus) doi: https://doi.org/10.1101/2020.01.31.929497, as well as publications relating to SARS-CoV, for example Hua, R., Zhou, Y., Wang, Y., Hua, Y and Tong, T. (2004) Identification of two antigenic epitopes on SARS-CoV spike protein, BBR 319, 929-935, wherein homologous epitopes may be found and SARS-CoV-2 epitopes be identified on account of their homology. For example, possible epitopes may be derived from SEQ ID NO5.

Variants may, in addition, comprise chemical modifications, for example labels such as isotopic labels or detectable labels or covalent modifications such as glycosylation, phosphorylation, acetylation, decarboxylation, citrullination, hydroxylation and the like. The person skilled in the art is familiar with methods for the modification of polypeptides. Moreover, variants may also be generated by way of fusion with other known polypeptides or variants thereof, for example artificial linkers, affinity tags, other antigens and the like. For example, SEQ ID NO3 is a fusion protein.

The variant of the polypeptide has biological activity. Such biological activity is the ability to bind to the respective antibody. It comprises an epitope having the ability or has itself the ability to bind to an IgA class antibody to SEQ ID NO1, preferably from a sample from a patient suffering from SARS-CoV-2, wherein more preferably the epitope comprises a sequence comprising at least 5, 6, 7 or 8 amino acid residues. More preferably, it does not bind specifically to homologues of SEQ ID NO1 from other coronaviruses, preferably from the group comprising MERS (SEQ ID NO6), NL63 (SEQ ID N010), 229E (SEQ ID NO7), OC43 (SEQ ID N08) and HKU1 (SEQ ID NO9), more preferably from the group comprising SARS-CoV-1 (SEQ ID NO11), MERS NL63, 229E, OC43 and HKU1.

The detection of the antibody for the diagnosis or methods according to the present invention comprises the use of a method selected from the group comprising immunodiffusion techniques, immunoelectrophoretic techniques, light scattering immunoassays, agglutination techniques, labeled immunoassays such as those from the group comprising radiolabeled immunoassays, enzyme immunoassays such as colorimetric assays, chemiluminscence immunoassays and immunofluorescence techniques. In a preferred embodiment, the complex is detected using a method selected from the group comprising immunodiffusion techniques, immunoelectrophoretic techniques, light scattering immunoassays, agglutination techniques, labeled immunoassays from the group comprising radiolabeled immunoassays, chemiluminscence immunoassays and immunofluorescence techniques. The person skilled in the art is familiar with these methods, which are also described in the state of the art, for example in Zane, H. D. (2001): Immunology - Theoretical & Practical Concepts in Laboratory Medicine, W. B. Saunders Company, in particular in Chapter 14. Preferably the test format is an ELISA and a microtiter plate comprising wells is used as a diagnostically useful carrier.

A secondary antibody is an antibody binding to all antibodies from an antibody class, preferably a human antibody class, preferably IgA and IgG and IgM antibodies, preferably IgA. Secondary antibodies typically recognize the constant domain of said class. A wide range of them is commercially available.

The diagnostically useful carrier is preferably selected from the group comprising a glass slide, preferably for microscopy, a biochip, a microtiter plate, a lateral flow device, a test strip, a membrane, preferably a line blot, a chromatography column and a bead, preferably a microtiter plate.

A polypeptide, preferably the polypeptide comprising SEQ ID NO1 or a variant thereof may be a recombinant protein, wherein the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", published by GE Healthcare Life Sciences, and in Burgess, R. R., Deutscher, M. P. (2009): Guide to Protein Purification). In another preferred embodiment, the polypeptide is an isolated polypeptide, wherein the term "isolated" means that the polypeptide has been enriched compared to its state upon production using a biotechnological or synthetic approach and is preferably pure, i.e. at least 60, 70, 80, 90, 95 or 99 percent of the polypeptide in the respective liquid consists of said polypeptide as judged by SDS polyacrylamide gel electrophoresis followed by Coomassie blue staining and visual inspection. Preferably any polypeptide on a carrier used as a means to capture an antibody is pure.

A secondary antibody comprising a detectable label may be used to detect IgA and IgG and IgM, preferably IgA class antibodies to SEQ ID NO1. In a preferred embodiment, the label is selected from the group comprising a fluorescent, a radioactive or an enzymatically active label, preferably one catalyzing a colorimetric reaction. FITC or another fluorescein derivative may be used as a fluorescent label. A protein having peroxidase activity may be used as an enzymatically active label. Preferably the secondary antibody recognizes mammalian, more preferably human antibodies. Preferably the secondary antibody is a monoclonal antibody.

Further described herein is a kit comprising a polypeptide comprising SEQ ID NO1 or a variant thereof, preferably coated to a diagnostically useful carrier, more preferably a microtiter plate, and one or more, preferably all reagents from the antibodies, wherein the secondary antibody may comprise a detectable label, a sample buffer, a detection solution, preferably a chromogen/substrate solution, a stop solution and a protective foil.

A calibrator is a reagent that binds to a polypeptide comprising SEQ ID NO1 or a variant thereof and is preferably recognized by secondary antibodies recognizing IgA class antibodies. The calibrator may be an IgA antibody to SEQ ID NO1 A positive control is a solution comprising a compound such as antibody to SEQ ID NO1, preferably from the group comprising IgA, IgG and IgM class antibodies, more preferably IgA, from the sample of a patient suffering from SARS-coV-2 at an amount that a positive result is obtained using the method according to the present invention. A negative control is a reagent that lacks such a compound and could comprise serum from a healthy person. A wash buffer may be used to wash the microtiter plate after the incubation to remove unspecific antibodies and could be PBS. The means for detecting the presence of an antibody could be a secondary antibody binding to the antibody class to be detected, preferably human IgA class antibodies, and is labeled, preferably with an enzyme, more preferably with an enzyme having peroxidase activity. The sample buffer may be used to dilute patient sample and may be PBS. The detection solution may yield a signal in the presence of the labeled secondary antibody and is preferably a color-developing solution and more preferably 3,3',5,5' tetramethylbenzidine/H2O2. The stop solution may be added to a reaction to stop the reaction of the detection solution and may comprise a strong acid, preferably 0.5 M sulphuric acid. The protective foil may be place on top of the microtiter plate to avoid evaporation.

The present specification comprises a range of novel nucleic acid and polypeptide sequences, more specifically
**SEQ ID NO1 (SARS-CoV-2 S1 Spike-Protein)**
**SEQ ID NO2 (SARS-CoV-2 Spike-Protein, C-terminally his-tagged, as expressed in cells for the example)**
**SEQ ID NO3 (SARS-CoV-2 Spike-Protein, C-terminally his-tagged, as after cleavage of signal peptide, used in the examples)**
**SEQ ID NO4 (SARS-CoV-2 Spike-Protein, nucleotide sequence encoding SEQ ID NO2)**
**SEQ ID NO5 (possible epitope)**
   NLKPFERDISTE
**SEQ ID NO6 (S1[MERS_CoV])**
**SEQ ID NO7 (S1[HCoV-229E])**
**SEQ ID NO8 (S1[HCoV-OC43])**
**SEQ ID NO9 (S1[HCoV-HKU1])**
**SEQ ID NO10 (S1[HCoV-NL63])**
**SEQ ID NO11 (S1[SARS_CoV])**
**SEQ ID NO12 (fragment of SEQ ID NO1)**

The present invention is further illustrated by the following examples, sequences and figures from which further features, embodiments, aspects and advantages of the present invention may be taken. All methods and materials similar or equivalent to those described herein can be used in the practice or testing of the present invention, with suitable methods and materials being described herein.

### Examples

### Samples

Eight samples from patients tested SARS-coV-2 positive by PCR as described by Corman *et al.* (Corman et al. (2020) Diagnostic detection of 2019-nCoV by real-time RT-PCR, https://www.who.int/docs/default-source/coronaviruse/protocol-v2-1.pdf?sfvrsn=a9ef618c 2), which were obtained 6 to 14 days after the infection and 14 samples from such patients obtained at an earlier time point after the infection were available.

In addition, a range of samples containing various coronaviruses was available, including 18 samples from patients infected with MERS, three samples from patients infected with SARS-CoV-1, four patients with NL63, three patients with 229E, six patients with OC43 and three patients with HKU1.

### Preparation of microtiter plates coated with antigen

SEQID NO2 was expressed in HEK293T cells using standard cloning of SEQ ID NO4 into the pTriEx-1 plasmid with an artificial signal sequence and a C-terminal His tag, resulting in the expression of SEQ ID NO2 and, after removal of the signal peptide, SEQ ID NO3. Transfected cells were cultures at 37°C and 8.5% CO2 in Dulbecco's modified eagle's medium with 10% fetal calf serum, 100 U/ml penicillin and 0.1 mg/ml streptomycin for three to five days. Cells were harvested, resuspended in 20 mM Tris-HCI pH 7.4, 10% (w/v) sucrose, 5 mM EDTA, 1 mM PMSF and stored at -80°C until further use.

To prepare SEQ ID NO3, cell culture supernatant was adjusted to 5 mmol/I tris chloride pH 8.0, 164 mmol/I sodium chloride, 50 mmol/I magnesium chloride, 20 mmol/I imidazole, 0,1% Triton X-100, cleared by centrifugation for 30 minutes at 17,600xg, 4°C, applied to Nickel Rapid Run (Agarose Bead Technologies, Miami, FL, USA) equilibrated with 5 mmol/I tris chloride pH 8.0, 300 mmol/I sodium chloride, 20 mmol/I imidazole and eluted by increasing the imidazole concentration to 150 mmol/I. All fractions containing SEQ ID NO3 were pooled and concentrated by ultrafiltration (VivaSpin, Sartorius, Gottingen, Germany). The final preparation was stored at -80°C until further use.

The final protein preparation of SEQ ID NO3 was treated with or without 16 mmol/I dithiotreitol and incubated at 70°C or at room temperature for 10 minutes, followed by SDS gel electrophoresis and Coomassie staining.

Protein identity was verified by mass spectrometry.

For use in microtiter ELISA the purified protein was diluted in PBS to final concentrations of approximately 1.5 µg/ml and used to coat ELISA microtiter plates (Nunc, Roskilde, Denmark) overnight.

### Experimental procedure

Samples were diluted 1:101 in IgG sample buffer, applied to microtiter plates and incubated as described for commercial EUROIMMUN ELISA Test-Kits, using reagents commercially available (e.g. El 2260-9601 G/A). The manual of El 2260-9601 G/A was followed. In brief: 60 min at 37 °C; 3 washing steps using washing buffer; addition of 100 µl of peroxidase-labelled anti-human IgG conjugate (rabbit) or anti-human IgA conjugate (rabbit) per well; incubation for 30 min at 37 °C; 3 washing steps using EUROIMMUN washing buffer; addition of 100 µl of chromogen/substrate solution (TMB/H₂O₂) per well; incubation for 30 min at room temperature; addition of 100 µl stop-solution (0.5 M sulfuric acid); measurement of optical density at 450 nm against 630 nm as a reference.

Calibration was carried out using commercially available calibrators (product number El 2606-9601 A, EUROIMMUN Medizinische Labordiagnostika AG). A ratio was calculated by dividing extinction of the control or patient sample by the extinction of the calibrator. Results below 0.8 were considered negative, results between 0.8 and 1.1 borderline, and results of more than 1.1 positive.

### Results

The primary data are shown in Table 1:

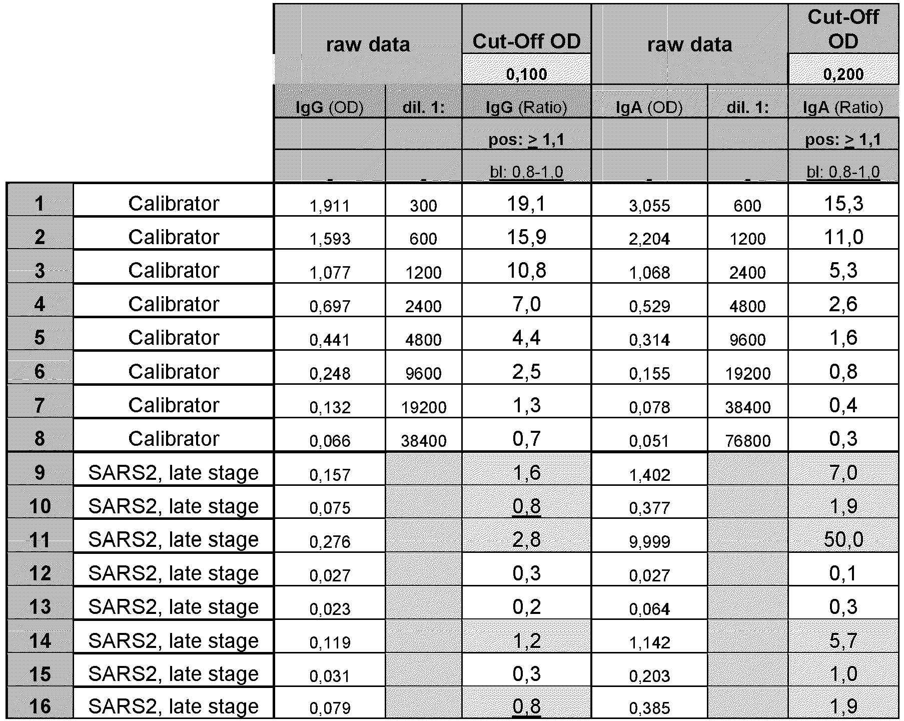

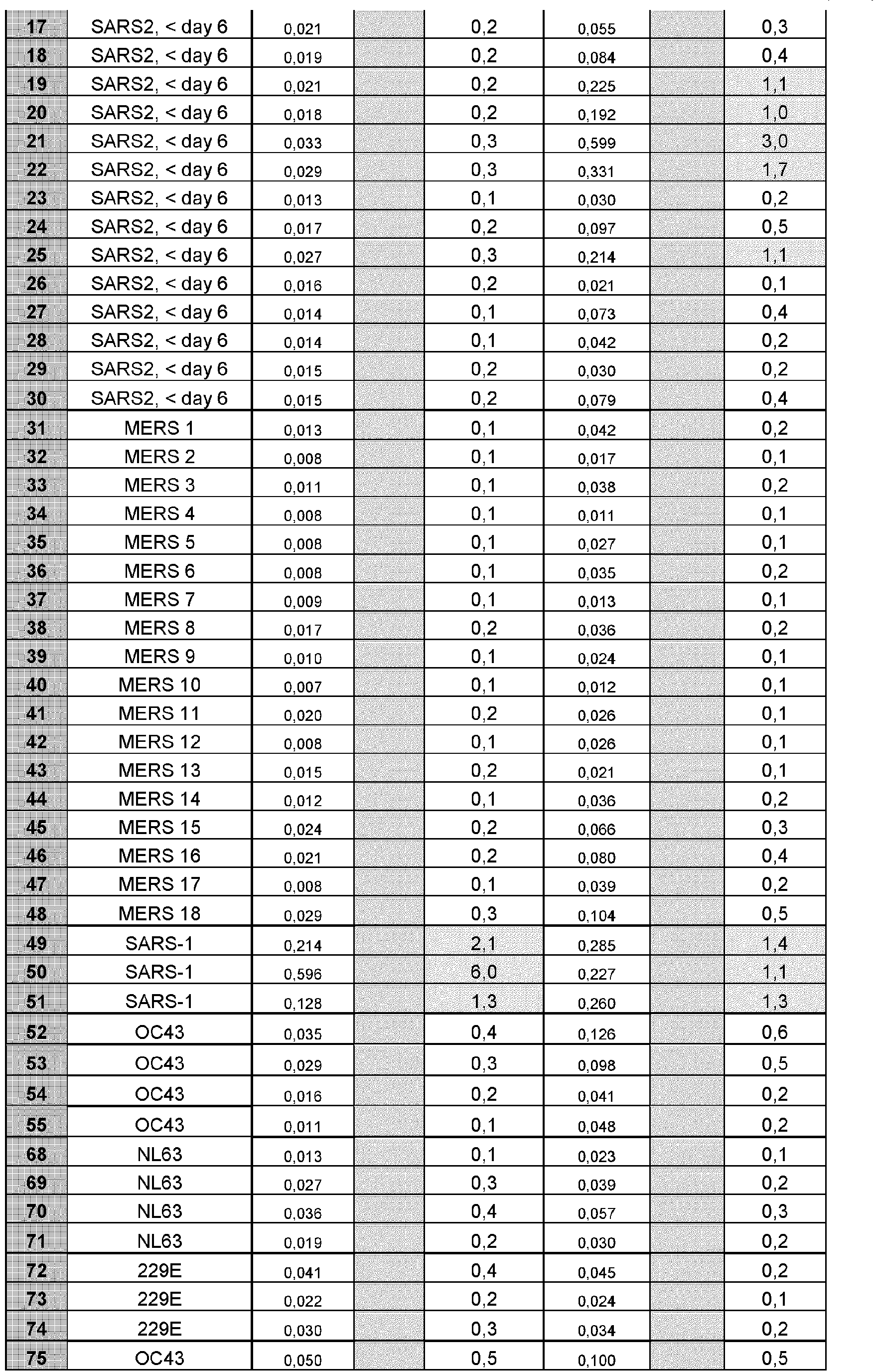

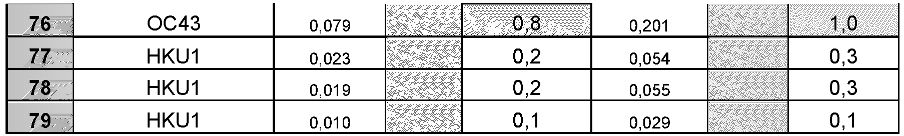

### Conclusions

The results show that antibodies to SEQ ID NO1 may be used for aiding in the diagnosis of an SARS-CoV-2 infection in samples from human patients.

Comparison of the data obtained with secondary antibodies recognizing IgG and IgA class antibodies shows that the detection of IgA antibodies is more sensitive: 4/14 patient samples taken at an earlier stage of the infection, before six days post onset of illness, could be correctly identified as positive when IgA class antibodies were detected, while the detection of IgG in the same samples gave negative results.

Both assays showed cross-reactivity with samples from SARS-CoV-1 patients, but virtually none of the samples from patients infected with MERS, NL63, 229E, OC43 and HKU1.

## Claims

1. A method for aiding in the diagnosis of a SARS-CoV-2 infection comprising the step of detecting the presence or absence of an IgA class antibody to SEQ ID NO1 in a blood sample from a subject.

2. The method according to claim 1, wherein the presence of an IgG and/or IgM class antibody to SEQ ID NO1 is detected in addition to an IgA class antibody to SEQ ID NO1.

3. The method according to any of claims 1 to 2, wherein the sample is selected from the group comprising whole blood, serum or plasma.

4. The method according to any of claims 1 to 3, wherein an IgA class antibody to SEQ ID NO1 is detected using a labeled secondary antibody binding to IgA class antibodies, preferably human IgA class antibodies.

5. The method according to any of claims 1 to 4, wherein the IgA antibody is detected using a method selected from the group comprising colorimetry, immunofluorescence, detection of enzymatic activity, chemiluminscence and radioactivity.

6. The method according to any of claims 1 to 5, wherein the infection is detected at an early stage, which is 5 or fewer days after the onset of disease symptoms.

7. A use of an IgA class antibody to SEQ ID NO1 for aiding in the diagnosis of a SARS-CoV-2 infection at an early stage, which is 5 or fewer days after the onset of disease symptoms,
wherein a subject is likely to suffer from a SARS-CoV-2 infection if an IgA class antibody to SEQ ID NO1 is detected in a blood sample from the subject.

8. The method according to any of claims 1 to 6, wherein the sample is a mammalian, preferably a human sample.

## Patentansprüche

1. Verfahren zum Unterstützen der Diagnose einer SARS-CoV-2-Infektion, umfassend den Schritt Nachweisen der Anwesenheit oder Abwesenheit eines Antikörpers gegen SEQ ID NO1 der Klasse IgA in einer Blutprobe von einem Subjekt.

2. Verfahren nach Anspruch 1, wobei die Anwesenheit eines Antikörpers gegen SEQ ID NO1 der Klasse IgG und/oder der Klasse IgM zusätzlich zu der eines Antikörpers der Klasse IgA nachgewiesen wird.

3. Verfahren nach einem der Ansprüche 1 bis 2, wobei die Probe aus der Gruppe ausgewählt ist, die Vollblut, Serum oder Plasma enthält.

4. Verfahren nach einem der Ansprüche 1 bis 3, wobei ein Antikörper gegen SEQ ID NO1 der Klasse IgA unter Verwendung eines markierten sekundären Antikörpers nachgewiesen wird, der gegen Antikörper der Klasse IgA bindet, bevorzugt gegen menschliche Antikörper der Klasse IgA.

5. Verfahren nach einem der Ansprüche 1 bis 4, wobei der Antikörper der Klasse IgA unter Verwendung eines Verfahrens ausgewählt aus der Gruppe umfassend Kolorimetrie, Immunfluoreszenz, Nachweis enzymatischer Aktivität, Nachweis von Chemilumineszenz und Nachweis von Radioaktivität nachgewiesen wird.

6. Verfahren nach einem der Ansprüche 1 bis 5, wobei die Infektion in einem frühen Stadium nachgewiesen wird, nämlich fünf oder weniger als fünf Tage nach Ausbruch der Krankheitssymptome.

7. Verwendung eines Antikörpers gegen SEQ ID NO1 der Klasse IgA zum Unterstützen der Diagnose einer SARS-CoV-2-Infektion in einem frühen Stadium, nämlich fünf oder weniger als fünf Tage nach Ausbruch der Krankheitssymptome,
wobei das Subjekt wahrscheinlich an einer SARS-CoV-2-Infektion leidet, wenn ein Antikörper gegen SEQ ID NO1 der Klasse IgA in einer Blutprobe von dem Subjekt nachgewiesen wird.

8. Verfahren nach einem der Ansprüche 1 bis 6, wobei die Probe von einem Säugetier, bevorzugt von einem Menschen stammt.

## Revendications

1. Procédé d'aide au diagnostic d'une infection à SARS-CoV-2 comprenant l'étape de détection de la présence ou l'absence d'un anticorps de classe IgA contre SEQ ID NO 1, dans un échantillon de sang provenant d'un sujet.

2. Procédé selon la revendication 1, dans lequel la présence d'un anticorps de classe IgG et/ou IgM contre SEQ ID NO 1 est détectée en plus d'un anticorps de classe IgA contre SEQ ID NO 1.

3. Procédé selon l'une quelconque des revendications 1 à 2, dans lequel l'échantillon est choisi dans le groupe comprenant du sang total, du sérum ou du plasma.

4. Procédé selon l'une quelconque des revendications 1 à 3, dans lequel un anticorps de classe IgA contre SEQ ID NO 1 est détecté au moyen d'un anticorps secondaire marqué, de préférence se liant à des anticorps de classe IgA, de préférence des anticorps de classe IgA humains.

5. Procédé selon l'une quelconque des revendications 1 à 4, dans lequel l'anticorps IgA est détecté au moyen d'un procédé choisi dans le groupe comprenant la colorimétrie, l'immunofluorescence, la détection d'une activité enzymatique, la chimiluminescence et la radioactivité.

6. Procédé selon l'une quelconque des revendications 1 à 5, dans lequel l'infection est détectée à un stade précoce, qui est 5 jours ou moins après l'apparition des symptômes de la maladie.

7. Utilisation d'un anticorps de classe IgA contre SEQ ID NO 1 pour l'aide au diagnostic d'une infection à SARS-CoV-2 à un stade précoce, qui est 5 jours ou moins après l'apparition des symptômes de la maladie,
dans lequel il est probable qu'un sujet souffre d'une infection à SARS-CoV-2 si un anticorps de classe IgA contre SEQ ID NO 1 est détecté dans un échantillon de sang provenant du sujet.

8. Procédé selon l'une quelconque des revendications 1 à 6, dans lequel l'échantillon est un échantillon de mammifère, de préférence humain.


### SEQUENCE LISTING

<110> EUROIMMUN Medizinische Labordiagnostika AG
<120> A method and reagents for the diagnosis of SARS-CoV-2
<130> 20PP007EP
<160> 12
<170> PatentIn version 3.5
<210> 1
   <211> 670
   <212> PRT
   <213> Artificial Sequence
<220>
   <223> SARS-CoV-2 S1 Spike-Protein
<400> 1
   Val Asn Leu Thr Thr Arg Thr Gln Leu Pro Pro Ala Tyr Thr Asn Ser
   1               5                   10                  15
   Phe Thr Arg Gly Val Tyr Tyr Pro Asp Lys Val Phe Arg Ser Ser Val
               20                  25                  30
   Leu His Ser Thr Gln Asp Leu Phe Leu Pro Phe Phe Ser Asn Val Thr
           35                  40                  45
   Trp Phe His Ala Ile His Val Ser Gly Thr Asn Gly Thr Lys Arg Phe
       50                  55                  60
   Asp Asn Pro Val Leu Pro Phe Asn Asp Gly Val Tyr Phe Ala Ser Thr
   65                  70                  75                  80
   Glu Lys Ser Asn Ile Ile Arg Gly Trp Ile Phe Gly Thr Thr Leu Asp
                   85                  90                  95
   Ser Lys Thr Gln Ser Leu Leu Ile Val Asn Asn Ala Thr Asn Val Val
               100                 105                 110
   Ile Lys Val Cys Glu Phe Gln Phe Cys Asn Asp Pro Phe Leu Gly Val
           115                 120                 125
   Tyr Tyr His Lys Asn Asn Lys Ser Trp Met Glu Ser Glu Phe Arg Val
       130                 135                 140
   Tyr Ser Ser Ala Asn Asn Cys Thr Phe Glu Tyr Val Ser Gln Pro Phe
   145                 150                 155                 160
   Leu Met Asp Leu Glu Gly Lys Gln Gly Asn Phe Lys Asn Leu Arg Glu
   165 170 175
   Phe Val Phe Lys Asn Ile Asp Gly Tyr Phe Lys Ile Tyr Ser Lys His
   180 185 190
   Thr Pro Ile Asn Leu Val Arg Asp Leu Pro Gln Gly Phe Ser Ala Leu
   195 200 205
   Glu Pro Leu Val Asp Leu Pro Ile Gly Ile Asn Ile Thr Arg Phe Gln
   210 215 220
   Thr Leu Leu Ala Leu His Arg Ser Tyr Leu Thr Pro Gly Asp Ser Ser
   225 230 235 240
   Ser Gly Trp Thr Ala Gly Ala Ala Ala Tyr Tyr Val Gly Tyr Leu Gln
   245 250 255
   Pro Arg Thr Phe Leu Leu Lys Tyr Asn Glu Asn Gly Thr Ile Thr Asp
   260 265 270
   Ala Val Asp Cys Ala Leu Asp Pro Leu Ser Glu Thr Lys Cys Thr Leu
   275 280 285
   Lys Ser Phe Thr Val Glu Lys Gly Ile Tyr Gln Thr Ser Asn Phe Arg
   290 295 300
   Val Gln Pro Thr Glu Ser Ile Val Arg Phe Pro Asn Ile Thr Asn Leu
   305 310 315 320
   Cys Pro Phe Gly Glu Val Phe Asn Ala Thr Arg Phe Ala Ser Val Tyr
   325 330 335
   Ala Trp Asn Arg Lys Arg Ile Ser Asn Cys Val Ala Asp Tyr Ser Val
   340 345 350
   Leu Tyr Asn Ser Ala Ser Phe Ser Thr Phe Lys Cys Tyr Gly Val Ser
   355 360 365
   Pro Thr Lys Leu Asn Asp Leu Cys Phe Thr Asn Val Tyr Ala Asp Ser
   370 375 380
   Phe Val Ile Arg Gly Asp Glu Val Arg Gln Ile Ala Pro Gly Gln Thr
   385 390 395 400
   Gly Lys Ile Ala Asp Tyr Asn Tyr Lys Leu Pro Asp Asp Phe Thr Gly
   405 410 415
   Cys Val Ile Ala Trp Asn Ser Asn Asn Leu Asp Ser Lys Val Gly Gly
   420 425 430
   Asn Tyr Asn Tyr Leu Tyr Arg Leu Phe Arg Lys Ser Asn Leu Lys Pro
   435 440 445
   Phe Glu Arg Asp Ile Ser Thr Glu Ile Tyr Gln Ala Gly Ser Thr Pro
   450 455 460
   Cys Asn Gly Val Glu Gly Phe Asn Cys Tyr Phe Pro Leu Gln Ser Tyr
   465 470 475 480
   Gly Phe Gln Pro Thr Asn Gly Val Gly Tyr Gln Pro Tyr Arg Val Val
   485 490 495
   Val Leu Ser Phe Glu Leu Leu His Ala Pro Ala Thr Val Cys Gly Pro
   500 505 510
   Lys Lys Ser Thr Asn Leu Val Lys Asn Lys Cys Val Asn Phe Asn Phe
   515 520 525
   Asn Gly Leu Thr Gly Thr Gly Val Leu Thr Glu Ser Asn Lys Lys Phe
   530 535 540
   Leu Pro Phe Gln Gln Phe Gly Arg Asp Ile Ala Asp Thr Thr Asp Ala
   545 550 555 560
   Val Arg Asp Pro Gln Thr Leu Glu Ile Leu Asp Ile Thr Pro Cys Ser
   565 570 575
   Phe Gly Gly Val Ser Val Ile Thr Pro Gly Thr Asn Thr Ser Asn Gln
   580 585 590
   Val Ala Val Leu Tyr Gln Asp Val Asn Cys Thr Glu Val Pro Val Ala
   595 600 605
   Ile His Ala Asp Gln Leu Thr Pro Thr Trp Arg Val Tyr Ser Thr Gly
   610 615 620
   Ser Asn Val Phe Gln Thr Arg Ala Gly Cys Leu Ile Gly Ala Glu His
   625 630 635 640
   Val Asn Asn Ser Tyr Glu Cys Asp Ile Pro Ile Gly Ala Gly Ile Cys
   645 650 655
   Ala Ser Tyr Gln Thr Gln Thr Asn Ser Pro Arg Arg Ala Arg
   660 665 670
<210> 2
   <211> 695
   <212> PRT
   <213> Artificial Sequence
<220>
   <223> SARS-CoV-2 Spike-Protein, C-terminally his-tagged
<400> 2
   Met Phe Val Phe Leu Val Leu Leu Pro Leu Val Ser Ser Gln Cys Val
   1               5                   10                  15
   Asn Leu Thr Thr Arg Thr Gln Leu Pro Pro Ala Tyr Thr Asn Ser Phe
               20                  25                  30
   Thr Arg Gly Val Tyr Tyr Pro Asp Lys Val Phe Arg Ser Ser Val Leu
           35                  40                  45
   His Ser Thr Gln Asp Leu Phe Leu Pro Phe Phe Ser Asn Val Thr Trp
       50                  55                  60
   Phe His Ala Ile His Val Ser Gly Thr Asn Gly Thr Lys Arg Phe Asp
   65                  70                  75                  80
   Asn Pro Val Leu Pro Phe Asn Asp Gly Val Tyr Phe Ala Ser Thr Glu
                   85                  90                  95
   Lys Ser Asn Ile Ile Arg Gly Trp Ile Phe Gly Thr Thr Leu Asp Ser
               100                 105                 110
   Lys Thr Gln Ser Leu Leu Ile Val Asn Asn Ala Thr Asn Val Val Ile
           115                 120                 125
   Lys Val Cys Glu Phe Gln Phe Cys Asn Asp Pro Phe Leu Gly Val Tyr
       130                 135                 140
   Tyr His Lys Asn Asn Lys Ser Trp Met Glu Ser Glu Phe Arg Val Tyr
   145                 150                 155                 160
   Ser Ser Ala Asn Asn Cys Thr Phe Glu Tyr Val Ser Gln Pro Phe Leu
                   165                 170                 175
   Met Asp Leu Glu Gly Lys Gln Gly Asn Phe Lys Asn Leu Arg Glu Phe
               180                 185                 190
   Val Phe Lys Asn Ile Asp Gly Tyr Phe Lys Ile Tyr Ser Lys His Thr
           195                 200                 205
   Pro Ile Asn Leu Val Arg Asp Leu Pro Gln Gly Phe Ser Ala Leu Glu
       210                 215                 220
   Pro Leu Val Asp Leu Pro Ile Gly Ile Asn Ile Thr Arg Phe Gln Thr
   225                 230                 235                 240
   Leu Leu Ala Leu His Arg Ser Tyr Leu Thr Pro Gly Asp Ser Ser Ser
                   245                 250                 255
   Gly Trp Thr Ala Gly Ala Ala Ala Tyr Tyr Val Gly Tyr Leu Gln Pro
               260                 265                 270
   Arg Thr Phe Leu Leu Lys Tyr Asn Glu Asn Gly Thr Ile Thr Asp Ala
           275                 280                 285
   Val Asp Cys Ala Leu Asp Pro Leu Ser Glu Thr Lys Cys Thr Leu Lys
       290                 295                 300
   Ser Phe Thr Val Glu Lys Gly Ile Tyr Gln Thr Ser Asn Phe Arg Val
   305                 310                 315                 320
   Gln Pro Thr Glu Ser Ile Val Arg Phe Pro Asn Ile Thr Asn Leu Cys
                   325                 330                 335
   Pro Phe Gly Glu Val Phe Asn Ala Thr Arg Phe Ala Ser Val Tyr Ala
               340                 345                 350
   Trp Asn Arg Lys Arg Ile Ser Asn Cys Val Ala Asp Tyr Ser Val Leu
           355                 360                 365
   Tyr Asn Ser Ala Ser Phe Ser Thr Phe Lys Cys Tyr Gly Val Ser Pro
       370                 375                 380
   Thr Lys Leu Asn Asp Leu Cys Phe Thr Asn Val Tyr Ala Asp Ser Phe
   385                 390                 395                 400
   Val Ile Arg Gly Asp Glu Val Arg Gln Ile Ala Pro Gly Gln Thr Gly
                   405                 410                 415
   Lys Ile Ala Asp Tyr Asn Tyr Lys Leu Pro Asp Asp Phe Thr Gly Cys
               420                 425                 430
   Val Ile Ala Trp Asn Ser Asn Asn Leu Asp Ser Lys Val Gly Gly Asn
           435                 440                 445
   Tyr Asn Tyr Leu Tyr Arg Leu Phe Arg Lys Ser Asn Leu Lys Pro Phe
       450                 455                 460
   Glu Arg Asp Ile Ser Thr Glu Ile Tyr Gln Ala Gly Ser Thr Pro Cys
   465                 470                 475                 480
   Asn Gly Val Glu Gly Phe Asn Cys Tyr Phe Pro Leu Gln Ser Tyr Gly
                   485                 490                 495
   Phe Gln Pro Thr Asn Gly Val Gly Tyr Gln Pro Tyr Arg Val Val Val
               500                 505                 510
   Leu Ser Phe Glu Leu Leu His Ala Pro Ala Thr Val Cys Gly Pro Lys
           515                 520                 525
   Lys Ser Thr Asn Leu Val Lys Asn Lys Cys Val Asn Phe Asn Phe Asn
       530                 535                 540
   Gly Leu Thr Gly Thr Gly Val Leu Thr Glu Ser Asn Lys Lys Phe Leu
   545                 550                 555                 560
   Pro Phe Gln Gln Phe Gly Arg Asp Ile Ala Asp Thr Thr Asp Ala Val
                   565                 570                 575
   Arg Asp Pro Gln Thr Leu Glu Ile Leu Asp Ile Thr Pro Cys Ser Phe
               580                 585                 590
   Gly Gly Val Ser Val Ile Thr Pro Gly Thr Asn Thr Ser Asn Gln Val
           595                 600                 605
   Ala Val Leu Tyr Gln Asp Val Asn Cys Thr Glu Val Pro Val Ala Ile
       610                 615                 620
   His Ala Asp Gln Leu Thr Pro Thr Trp Arg Val Tyr Ser Thr Gly Ser
   625                 630                 635                 640
   Asn Val Phe Gln Thr Arg Ala Gly Cys Leu Ile Gly Ala Glu His Val
                   645                 650                 655
   Asn Asn Ser Tyr Glu Cys Asp Ile Pro Ile Gly Ala Gly Ile Cys Ala
               660                 665                 670
   Ser Tyr Gln Thr Gln Thr Asn Ser Pro Arg Arg Ala Arg Leu Glu His
           675                 680                 685
   His His His His His His His
       690                 695
<210> 3
   <211> 680
   <212> PRT
   <213> Artificial Sequence
<220>
   <223> SARS-CoV-2 Spike-Protein, C-terminally his-tagged, as after cleavage of signal peptide
<400> 3
   Val Asn Leu Thr Thr Arg Thr Gln Leu Pro Pro Ala Tyr Thr Asn Ser
   1               5                   10                  15
   Phe Thr Arg Gly Val Tyr Tyr Pro Asp Lys Val Phe Arg Ser Ser Val
               20                  25                  30
   Leu His Ser Thr Gln Asp Leu Phe Leu Pro Phe Phe Ser Asn Val Thr
           35                  40                  45
   Trp Phe His Ala Ile His Val Ser Gly Thr Asn Gly Thr Lys Arg Phe
       50                  55                  60
   Asp Asn Pro Val Leu Pro Phe Asn Asp Gly Val Tyr Phe Ala Ser Thr
   65                  70                  75                  80
   Glu Lys Ser Asn Ile Ile Arg Gly Trp Ile Phe Gly Thr Thr Leu Asp
                   85                  90                  95
   Ser Lys Thr Gln Ser Leu Leu Ile Val Asn Asn Ala Thr Asn Val Val
               100                 105                 110
   Ile Lys Val Cys Glu Phe Gln Phe Cys Asn Asp Pro Phe Leu Gly Val
           115                 120                 125
   Tyr Tyr His Lys Asn Asn Lys Ser Trp Met Glu Ser Glu Phe Arg Val
       130                 135                 140
   Tyr Ser Ser Ala Asn Asn Cys Thr Phe Glu Tyr Val Ser Gln Pro Phe
   145                 150                 155                 160
   Leu Met Asp Leu Glu Gly Lys Gln Gly Asn Phe Lys Asn Leu Arg Glu
                   165                 170                 175
   Phe Val Phe Lys Asn Ile Asp Gly Tyr Phe Lys Ile Tyr Ser Lys His
               180                 185                 190
   Thr Pro Ile Asn Leu Val Arg Asp Leu Pro Gln Gly Phe Ser Ala Leu
           195                 200                 205
   Glu Pro Leu Val Asp Leu Pro Ile Gly Ile Asn Ile Thr Arg Phe Gln
       210                 215                 220
   Thr Leu Leu Ala Leu His Arg Ser Tyr Leu Thr Pro Gly Asp Ser Ser
   225                 230                 235                 240
   Ser Gly Trp Thr Ala Gly Ala Ala Ala Tyr Tyr Val Gly Tyr Leu Gln
                   245                 250                 255
   Pro Arg Thr Phe Leu Leu Lys Tyr Asn Glu Asn Gly Thr Ile Thr Asp
               260                 265                 270
   Ala Val Asp Cys Ala Leu Asp Pro Leu Ser Glu Thr Lys Cys Thr Leu
           275                 280                 285
   Lys Ser Phe Thr Val Glu Lys Gly Ile Tyr Gln Thr Ser Asn Phe Arg
       290                 295                 300
   Val Gln Pro Thr Glu Ser Ile Val Arg Phe Pro Asn Ile Thr Asn Leu
   305                 310                 315                 320
   Cys Pro Phe Gly Glu Val Phe Asn Ala Thr Arg Phe Ala Ser Val Tyr
                   325                 330                 335
   Ala Trp Asn Arg Lys Arg Ile Ser Asn Cys Val Ala Asp Tyr Ser Val
               340                 345                 350
   Leu Tyr Asn Ser Ala Ser Phe Ser Thr Phe Lys Cys Tyr Gly Val Ser
           355                 360                 365
   Pro Thr Lys Leu Asn Asp Leu Cys Phe Thr Asn Val Tyr Ala Asp Ser
       370                 375                 380
   Phe Val Ile Arg Gly Asp Glu Val Arg Gln Ile Ala Pro Gly Gln Thr
   385                 390                 395                 400
   Gly Lys Ile Ala Asp Tyr Asn Tyr Lys Leu Pro Asp Asp Phe Thr Gly
                   405                 410                 415
   Cys Val Ile Ala Trp Asn Ser Asn Asn Leu Asp Ser Lys Val Gly Gly
               420                 425                 430
   Asn Tyr Asn Tyr Leu Tyr Arg Leu Phe Arg Lys Ser Asn Leu Lys Pro
           435                 440                 445
   Phe Glu Arg Asp Ile Ser Thr Glu Ile Tyr Gln Ala Gly Ser Thr Pro
       450                 455                 460
   Cys Asn Gly Val Glu Gly Phe Asn Cys Tyr Phe Pro Leu Gln Ser Tyr
   465 470 475 480
   Gly Phe Gln Pro Thr Asn Gly Val Gly Tyr Gln Pro Tyr Arg Val Val
   485 490 495
   Val Leu Ser Phe Glu Leu Leu His Ala Pro Ala Thr Val Cys Gly Pro
   500 505 510
   Lys Lys Ser Thr Asn Leu Val Lys Asn Lys Cys Val Asn Phe Asn Phe
   515 520 525
   Asn Gly Leu Thr Gly Thr Gly Val Leu Thr Glu Ser Asn Lys Lys Phe
   530 535 540
   Leu Pro Phe Gln Gln Phe Gly Arg Asp Ile Ala Asp Thr Thr Asp Ala
   545 550 555 560
   Val Arg Asp Pro Gln Thr Leu Glu Ile Leu Asp Ile Thr Pro Cys Ser
   565 570 575
   Phe Gly Gly Val Ser Val Ile Thr Pro Gly Thr Asn Thr Ser Asn Gln
   580 585 590
   Val Ala Val Leu Tyr Gln Asp Val Asn Cys Thr Glu Val Pro Val Ala
   595 600 605
   Ile His Ala Asp Gln Leu Thr Pro Thr Trp Arg Val Tyr Ser Thr Gly
   610 615 620
   Ser Asn Val Phe Gln Thr Arg Ala Gly Cys Leu Ile Gly Ala Glu His
   625 630 635 640
   Val Asn Asn Ser Tyr Glu Cys Asp Ile Pro Ile Gly Ala Gly Ile Cys
   645 650 655
   Ala Ser Tyr Gln Thr Gln Thr Asn Ser Pro Arg Arg Ala Arg Leu Glu
   660 665 670
   His His His His His His His His
   675 680
<210> 4
   <211> 2088
   <212> DNA
   <213> Artificial Sequence
<220>
   <223> SARS-CoV-2 Spike-Protein, nucleotide sequence encoding SEQ ID NO2
<400> 4
   atgttcgtat tccttgttct gctgcctttg gttagcagtc agtgtgtcaa cctgacaact     60
   cgcacgcaac tgccgccagc ttacaccaac tctttcacaa gaggcgtcta ctacccggac    120
   aaagtgtttc gctcatcagt gctgcactct acacaagatt tgtttctgcc attcttctct    180
   aacgtaacct ggtttcacgc gattcatgtg tctgggacaa atgggaccaa gcgcttcgac    240
   aaccccgtgc tgccattcaa tgacggggtg tattttgcct ccaccgagaa atccaatatc    300
   atccgaggat ggattttcgg tactacgctg gactctaaaa cgcagtctct cttgatcgtt    360
   aataacgcca caaatgttgt cattaaggtg tgcgagtttc agttctgtaa tgatcccttt    420
   ctgggtgtgt attaccacaa gaataacaag tcatggatgg aaagcgagtt tcgcgtgtac    480
   tcaagtgcca ataactgcac attcgagtat gtgtcccagc ctttcctgat ggatctcgaa    540
   ggcaaacagg ggaacttcaa gaatctgcgc gagttcgtgt ttaagaacat cgacggttat    600
   ttcaagatct acagcaaaca tacacccatt aacctggtca gggatctccc tcagggattc    660
   tccgccctgg aacccttggt ggacttgccc attgggatta acatcactag attccagacc    720
   ctgctggccc ttcaccgttc ctatcttact cctggcgaca gtagcagtgg atggaccgca    780
   ggagcagccg cttactatgt aggctatctg cagccacgga ccttcctcct caagtacaat    840
   gaaaatggta ccataactga tgctgtggac tgcgctctgg atccactctc cgaaactaaa    900
   tgcaccctta aaagcttcac ggtcgaaaag ggaatctacc agacaagtaa ctttcgggta    960
   caacccactg agtccatcgt gcggtttcct aacatcacaa atctctgccc ctttggtgaa   1020
   gtgtttaacg ccactaggtt cgcttctgtt tatgcgtgga atcggaagag gatttccaat   1080
   tgcgtggcag actactctgt cctgtataat agcgctagct tcagcacctt caaatgttac   1140
   ggggtaagcc caactaaact gaacgacctc tgttttacca acgtgtatgc cgatagcttt   1200
   gtcatacgag gagatgaggt tcgtcagatt gctcctggcc aaacggggaa aatcgcagac   1260
   tacaactaca agcttcccga cgacttcaca ggatgcgtga tcgcgtggaa ctcaaataat   1320
   ctggatagca aggttggtgg caattataac tacctgtatc gactgttcag gaaaagcaac   1380
   ctcaaaccct ttgagcgcga catcagcacc gagatatacc aagccggttc aacaccttgc   1440
   aatggggtgg aagggtttaa ctgctatttc ccacttcaga gctatgggtt tcagccaacc   1500
   aatggagtcg gctaccagcc ctatcgggtg gtagtcctgt cctttgagct gttgcatgcg   1560
   cctgccacag tctgtggccc taagaagagt acgaatctgg tgaagaacaa gtgcgtcaac   1620
   ttcaatttta acggcttgac tggaacagga gttctgaccg agtccaacaa gaaattcctt   1680
   ccttttcagc agtttggaag ggatatagcc gacactaccg atgccgttcg ggatccacag   1740
   acactggaga ttctggacat tactccgtgc tcatttggcg gtgtatctgt catcacacct   1800
   gggaccaata cctcaaatca ggtggctgtg ctctaccagg atgtgaattg taccgaagtt   1860
   ccagtggcaa ttcatgccga tcaactgact cccacctgga gagtgtacag tactggcagt      1920
   aacgtgtttc agacaagagc tggctgtctc ataggcgcag aacacgtcaa caacagctat      1980
   gagtgtgaca ttccgatcgg cgcaggcatc tgtgcatcct accagacgca aaccaactct      2040
   cccagaagag ccaggctcga gcaccaccat caccatcacc atcactaa                   2088
<210> 5
   <211> 12
   <212> PRT
   <213> Artificial Sequence
<220>
   <223> possible epitope
<400> 5
   Asn Leu Lys Pro Phe Glu Arg Asp Ile Ser Thr Glu
   1               5                   10
<210> 6
   <211> 734
   <212> PRT
   <213> Artificial Sequence
<220>
   <223> S1 [MERS_CoV]
<400> 6
   Tyr Val Asp Val Gly Pro Asp Ser Val Lys Ser Ala Cys Ile Glu Val
   1               5                   10                  15
   Asp Ile Gln Gln Thr Phe Phe Asp Lys Thr Trp Pro Arg Pro Ile Asp
               20                  25                  30
   Val Ser Lys Ala Asp Gly Ile Ile Tyr Pro Gln Gly Arg Thr Tyr Ser
           35                  40                  45
   Asn Ile Thr Ile Thr Tyr Gln Gly Leu Phe Pro Tyr Gln Gly Asp His
       50                  55                  60
   Gly Asp Met Tyr Val Tyr Ser Ala Gly His Ala Thr Gly Thr Thr Pro
   65                  70                  75                  80
   Gln Lys Leu Phe Val Ala Asn Tyr Ser Gln Asp Val Lys Gln Phe Ala
                   85                  90                  95
   Asn Gly Phe Val Val Arg Ile Gly Ala Ala Ala Asn Ser Thr Gly Thr
               100                 105                 110
   Val Ile Ile Ser Pro Ser Thr Ser Ala Thr Ile Arg Lys Ile Tyr Pro
           115                 120                 125
   Ala Phe Met Leu Gly Ser Ser Val Gly Asn Phe Ser Asp Gly Lys Met
       130                 135                 140
   Gly Arg Phe Phe Asn His Thr Leu Val Leu Leu Pro Asp Gly Cys Gly
   145                 150                 155                 160
   Thr Leu Leu Arg Ala Phe Tyr Cys Ile Leu Glu Pro Arg Ser Gly Asn
                   165                 170                 175
   His Cys Pro Ala Gly Asn Ser Tyr Thr Ser Phe Ala Thr Tyr His Thr
               180                 185                 190
   Pro Ala Thr Asp Cys Ser Asp Gly Asn Tyr Asn Arg Asn Ala Ser Leu
           195                 200                 205
   Asn Ser Phe Lys Glu Tyr Phe Asn Leu Arg Asn Cys Thr Phe Met Tyr
       210                 215                 220
   Thr Tyr Asn Ile Thr Glu Asp Glu Ile Leu Glu Trp Phe Gly Ile Thr
   225                 230                 235                 240
   Gln Thr Ala Gln Gly Val His Leu Phe Ser Ser Arg Tyr Val Asp Leu
                   245                 250                 255
   Tyr Gly Gly Asn Met Phe Gln Phe Ala Thr Leu Pro Val Tyr Asp Thr
               260                 265                 270
   Ile Lys Tyr Tyr Ser Ile Ile Pro His Ser Ile Arg Ser Ile Gln Ser
           275                 280                 285
   Asp Arg Lys Ala Trp Ala Ala Phe Tyr Val Tyr Lys Leu Gln Pro Leu
       290                 295                 300
   Thr Phe Leu Leu Asp Phe Ser Val Asp Gly Tyr Ile Arg Arg Ala Ile
   305                 310                 315                 320
   Asp Cys Gly Phe Asn Asp Leu Ser Gln Leu His Cys Ser Tyr Glu Ser
                   325                 330                 335
   Phe Asp Val Glu Ser Gly Val Tyr Ser Val Ser Ser Phe Glu Ala Lys
               340                 345                 350
   Pro Ser Gly Ser Val Val Glu Gln Ala Glu Gly Val Glu Cys Asp Phe
           355                 360                 365
   Ser Pro Leu Leu Ser Gly Thr Pro Pro Gln Val Tyr Asn Phe Lys Arg
   370 375 380
   Leu Val Phe Thr Asn Cys Asn Tyr Asn Leu Thr Lys Leu Leu Ser Leu
   385 390 395 400
   Phe Ser Val Asn Asp Phe Thr Cys Ser Gln Ile Ser Pro Ala Ala Ile
   405 410 415
   Ala Ser Asn Cys Tyr Ser Ser Leu Ile Leu Asp Tyr Phe Ser Tyr Pro
   420 425 430
   Leu Ser Met Lys Ser Asp Leu Ser Val Ser Ser Ala Gly Pro Ile Ser
   435 440 445
   Gln Phe Asn Tyr Lys Gln Ser Phe Ser Asn Pro Thr Cys Leu Ile Leu
   450 455 460
   Ala Thr Val Pro His Asn Leu Thr Thr Ile Thr Lys Pro Leu Lys Tyr
   465 470 475 480
   Ser Tyr Ile Asn Lys Cys Ser Arg Leu Leu Ser Asp Asp Arg Thr Glu
   485 490 495
   Val Pro Gln Leu Val Asn Ala Asn Gln Tyr Ser Pro Cys Val Ser Ile
   500 505 510
   Val Pro Ser Thr Val Trp Glu Asp Gly Asp Tyr Tyr Arg Lys Gln Leu
   515 520 525
   Ser Pro Leu Glu Gly Gly Gly Trp Leu Val Ala Ser Gly Ser Thr Val
   530 535 540
   Ala Met Thr Glu Gln Leu Gln Met Gly Phe Gly Ile Thr Val Gln Tyr
   545 550 555 560
   Gly Thr Asp Thr Asn Ser Val Cys Pro Lys Leu Glu Phe Ala Asn Asp
   565 570 575
   Thr Lys Ile Ala Ser Gln Leu Gly Asn Cys Val Glu Tyr Ser Leu Tyr
   580 585 590
   Gly Val Ser Gly Arg Gly Val Phe Gln Asn Cys Thr Ala Val Gly Val
   595 600 605
   Arg Gln Gln Arg Phe Val Tyr Asp Ala Tyr Gln Asn Leu Val Gly Tyr
   610 615 620
   Tyr Ser Asp Asp Gly Asn Tyr Tyr Cys Leu Arg Ala Cys Val Ser Val
   625 630 635 640
   Pro Val Ser Val Ile Tyr Asp Lys Glu Thr Lys Thr His Ala Thr Leu
   645 650 655
   Phe Gly Ser Val Ala Cys Glu His Ile Ser Ser Thr Met Ser Gln Tyr
   660 665 670
   Ser Arg Ser Thr Arg Ser Met Leu Lys Arg Arg Asp Ser Thr Tyr Gly
   675 680 685
   Pro Leu Gln Thr Pro Val Gly Cys Val Leu Gly Leu Val Asn Ser Ser
   690 695 700
   Leu Phe Val Glu Asp Cys Lys Leu Pro Leu Gly Gln Ser Leu Cys Ala
   705 710 715 720
   Leu Pro Asp Thr Pro Ser Thr Leu Thr Pro Arg Ser Val Arg
   725 730
<210> 7
   <211> 521
   <212> PRT
   <213> Artificial Sequence
<220>
   <223> S1 [HCoV-229E]
<400> 7
   Cys Gln Thr Thr Asn Gly Leu Asn Thr Ser Tyr Ser Val Cys Asn Gly
   1               5                   10                  15
   Cys Val Gly Tyr Ser Glu Asn Val Phe Ala Val Glu Ser Gly Gly Tyr
               20                  25                  30
   Ile Pro Ser Asp Phe Ala Phe Asn Asn Trp Phe Leu Leu Thr Asn Thr
           35                  40                  45
   Ser Ser Val Val Asp Gly Val Val Arg Ser Phe Gln Pro Leu Leu Leu
       50                  55                  60
   Asn Cys Leu Trp Ser Val Ser Gly Leu Arg Phe Thr Thr Gly Phe Val
   65                  70                  75                  80
   Tyr Phe Asn Gly Thr Gly Arg Gly Asp Cys Lys Gly Phe Ser Ser Asp
                   85                  90                  95
   Val Leu Ser Asp Val Ile Arg Tyr Asn Leu Asn Phe Glu Glu Asn Leu
   100 105 110
   Arg Arg Gly Thr Ile Leu Phe Lys Thr Ser Tyr Gly Val Val Val Phe
   115 120 125
   Tyr Cys Thr Asn Asn Thr Leu Val Ser Gly Asp Ala His Ile Pro Phe
   130 135 140
   Gly Thr Val Leu Gly Asn Phe Tyr Cys Phe Val Asn Thr Thr Ile Gly
   145 150 155 160
   Asn Glu Thr Thr Ser Ala Phe Val Gly Ala Leu Pro Lys Thr Val Arg
   165 170 175
   Glu Phe Val Ile Ser Arg Thr Gly His Phe Tyr Ile Asn Gly Tyr Arg
   180 185 190
   Tyr Phe Thr Leu Gly Asn Val Glu Ala Val Asn Phe Asn Val Thr Thr
   195 200 205
   Ala Glu Thr Thr Asp Phe Cys Thr Val Ala Leu Ala Ser Tyr Ala Asp
   210 215 220
   Val Leu Val Asn Val Ser Gln Thr Ser Ile Ala Asn Ile Ile Tyr Cys
   225 230 235 240
   Asn Ser Val Ile Asn Arg Leu Arg Cys Asp Gln Leu Ser Phe Asp Val
   245 250 255
   Pro Asp Gly Phe Tyr Ser Thr Ser Pro Ile Gln Ser Val Glu Leu Pro
   260 265 270
   Val Ser Ile Val Ser Leu Pro Val Tyr His Lys His Thr Phe Ile Val
   275 280 285
   Leu Tyr Val Asp Phe Lys Pro Gln Ser Gly Gly Gly Lys Cys Phe Asn
   290 295 300
   Cys Tyr Pro Ala Gly Val Asn Ile Thr Leu Ala Asn Phe Asn Glu Thr
   305 310 315 320
   Lys Gly Pro Leu Cys Val Asp Thr Ser His Phe Thr Thr Lys Tyr Val
   325 330 335
   Ala Val Tyr Ala Asn Val Gly Arg Trp Ser Ala Ser Ile Asn Thr Gly
   340 345 350
   Asn Cys Pro Phe Ser Phe Gly Lys Val Asn Asn Phe Val Lys Phe Gly
           355                 360                 365
   Ser Val Cys Phe Ser Leu Lys Asp Ile Pro Gly Gly Cys Ala Met Pro
       370                 375                 380
   Ile Val Ala Asn Trp Ala Tyr Ser Lys Tyr Tyr Thr Ile Gly Ser Leu
   385                 390                 395                 400
   Tyr Val Ser Trp Ser Asp Gly Asp Gly Ile Thr Gly Val Pro Gln Pro
                   405                 410                 415
   Val Glu Gly Val Ser Ser Phe Met Asn Val Thr Leu Asp Lys Cys Thr
               420                 425                 430
   Lys Tyr Asn Ile Tyr Asp Val Ser Gly Val Gly Val Ile Arg Val Ser
           435                 440                 445
   Asn Asp Thr Phe Leu Asn Gly Ile Thr Tyr Thr Ser Thr Ser Gly Asn
       450                 455                 460
   Leu Leu Gly Phe Lys Asp Val Thr Lys Gly Thr Ile Tyr Ser Ile Thr
   465                 470                 475                 480
   Pro Cys Asn Pro Pro Asp Gln Leu Val Val Tyr Gln Gln Ala Val Val
                   485                 490                 495
   Gly Ala Met Leu Ser Glu Asn Phe Thr Ser Tyr Gly Phe Ser Asn Val
               500                 505                 510
   Val Glu Leu Pro Lys Phe Phe Tyr Ala
           515                 520
<210> 8
   <211> 745
   <212> PRT
   <213> Artificial Sequence
<220>
   <223> S1 [HCoV-OC43]
<400> 8
   Ala Val Ile Gly Asp Leu Lys Cys Thr Ser Asp Asn Ile Asn Asp Lys
   1               5                   10                  15
   Asp Thr Gly Pro Pro Pro Ile Ser Thr Asp Thr Val Asp Val Thr Asn
               20                  25                  30
   Gly Leu Gly Thr Tyr Tyr Val Leu Asp Arg Val Tyr Leu Asn Thr Thr
               35                  40                  45
   Leu Phe Leu Asn Gly Tyr Tyr Pro Thr Ser Gly Ser Thr Tyr Arg Asn
       50                  55                  60
   Met Ala Leu Lys Gly Ser Val Leu Leu Ser Arg Leu Trp Phe Lys Pro
   65                  70                  75                  80
   Pro Phe Leu Ser Asp Phe Ile Asn Gly Ile Phe Ala Lys Val Lys Asn
                   85                  90                  95
   Thr Lys Val Ile Lys Asp Arg Val Met Tyr Ser Glu Phe Pro Ala Ile
               100                 105                 110
   Thr Ile Gly Ser Thr Phe Val Asn Thr Ser Tyr Ser Val Val Val Gln
           115                 120                 125
   Pro Arg Thr Ile Asn Ser Thr Gln Asp Gly Asp Asn Lys Leu Gln Gly
       130                 135                 140
   Leu Leu Glu Val Ser Val Cys Gln Tyr Asn Met Cys Glu Tyr Pro Gln
   145                 150                 155                 160
   Thr Ile Cys His Pro Asn Leu Gly Asn His Arg Lys Glu Leu Trp His
                   165                 170                 175
   Leu Asp Thr Gly Val Val Ser Cys Leu Tyr Lys Arg Asn Phe Thr Tyr
               180                 185                 190
   Asp Val Asn Ala Asp Tyr Leu Tyr Phe His Phe Tyr Gln Glu Gly Gly
           195                 200                 205
   Thr Phe Tyr Ala Tyr Phe Thr Asp Thr Gly Val Val Thr Lys Phe Leu
       210                 215                 220
   Phe Asn Val Tyr Leu Gly Met Ala Leu Ser His Tyr Tyr Val Met Pro
   225                 230                 235                 240
   Leu Thr Cys Asn Ser Lys Leu Thr Leu Glu Tyr Trp Val Thr Pro Leu
                   245                 250                 255
   Thr Ser Arg Gln Tyr Leu Leu Ala Phe Asn Gln Asp Gly Ile Ile Phe
               260                 265                 270
   Asn Ala Glu Asp Cys Met Ser Asp Phe Met Ser Glu Ile Lys Cys Lys
           275                 280                 285
   Thr Gln Ser Ile Ala Pro Pro Thr Gly Val Tyr Glu Leu Asn Gly Tyr
   290 295 300
   Thr Val Gln Pro Ile Ala Asp Val Tyr Arg Arg Lys Pro Asn Leu Pro
   305 310 315 320
   Asn Cys Asn Ile Glu Ala Trp Leu Asn Asp Lys Ser Val Pro Ser Pro
   325 330 335
   Leu Asn Trp Glu Arg Lys Thr Phe Ser Asn Cys Asn Phe Asn Met Ser
   340 345 350
   Ser Leu Met Ser Phe Ile Gln Ala Asp Ser Phe Thr Cys Asn Asn Ile
   355 360 365
   Asp Ala Ala Lys Ile Tyr Gly Met Cys Phe Ser Ser Ile Thr Ile Asp
   370 375 380
   Lys Phe Ala Ile Pro Asn Gly Arg Lys Val Asp Leu Gln Leu Gly Asn
   385 390 395 400
   Leu Gly Tyr Leu Gln Ser Phe Asn Tyr Arg Ile Asp Thr Thr Ala Thr
   405 410 415
   Ser Cys Gln Leu Tyr Tyr Asn Leu Pro Ala Ala Asn Val Ser Val Ser
   420 425 430
   Arg Phe Asn Pro Ser Thr Trp Asn Lys Arg Phe Gly Phe Ile Glu Asp
   435 440 445
   Ser Val Phe Lys Pro Arg Pro Ala Gly Val Leu Thr Asn His Asp Val
   450 455 460
   Val Tyr Ala Gln His Cys Phe Lys Ala Pro Lys Asn Phe Cys Pro Cys
   465 470 475 480
   Lys Leu Asn Gly Ser Cys Val Gly Ser Gly Pro Gly Lys Asn Asn Gly
   485 490 495
   Ile Gly Thr Cys Pro Ala Gly Thr Asn Tyr Leu Thr Cys Asp Asn Leu
   500 505 510
   Cys Thr Pro Asp Pro Ile Thr Phe Thr Gly Thr Tyr Lys Cys Pro Gln
   515 520 525
   Thr Lys Ser Leu Val Gly Ile Gly Glu His Cys Ser Gly Leu Ala Val
   530 535 540
   Lys Ser Asp Tyr Cys Gly Gly Asn Ser Cys Thr Cys Arg Pro Gln Ala
   545                 550                 555                 560
   Phe Leu Gly Trp Ser Ala Asp Ser Cys Leu Gln Gly Asp Lys Cys Asn
                   565                 570                 575
   Ile Phe Ala Asn Phe Ile Leu His Asp Val Asn Ser Gly Leu Thr Cys
               580                 585                 590
   Ser Thr Asp Leu Gln Lys Ala Asn Thr Asp Ile Ile Leu Gly Val Cys
           595                 600                 605
   Val Asn Tyr Asp Leu Tyr Gly Ile Leu Gly Gln Gly Ile Phe Val Glu
       610                 615                 620
   Val Asn Ala Thr Tyr Tyr Asn Ser Trp Gln Asn Leu Leu Tyr Asp Ser
   625                 630                 635                 640
   Asn Gly Asn Leu Tyr Gly Phe Arg Asp Tyr Ile Ile Asn Arg Thr Phe
                   645                 650                 655
   Met Ile Arg Ser Cys Tyr Ser Gly Arg Val Ser Ala Ala Phe His Ala
               660                 665                 670
   Asn Ser Ser Glu Pro Ala Leu Leu Phe Arg Asn Ile Lys Cys Asn Tyr
           675                 680                 685
   Val Phe Asn Asn Ser Leu Thr Arg Gln Leu Gln Pro Ile Asn Tyr Phe
       690                 695                 700
   Asp Ser Tyr Leu Gly Cys Val Val Asn Ala Tyr Asn Ser Thr Ala Ile
   705                 710                 715                 720
   Ser Val Gln Thr Cys Asp Leu Thr Val Gly Ser Gly Tyr Cys Val Asp
                   725                 730                 735
   Tyr Ser Lys Asn Arg Arg Ser Arg Gly
               740                 745
<210> 9
   <211> 744
   <212> PRT
   <213> Artificial Sequence
<220>
   <223> S1 [HCoV-HKU1]
<400> 9
   Ala Val Ile Gly Asp Phe Asn Cys Thr Asn Ser Phe Ile Asn Asp Tyr
   1               5                   10                  15
   Asn Lys Thr Ile Pro Arg Ile Ser Glu Asp Val Val Asp Val Ser Leu
               20                  25                  30
   Gly Leu Gly Thr Tyr Tyr Val Leu Asn Arg Val Tyr Leu Asn Thr Thr
           35                  40                  45
   Leu Leu Phe Thr Gly Tyr Phe Pro Lys Ser Gly Ala Asn Phe Arg Asp
       50                  55                  60
   Leu Ala Leu Lys Gly Ser Ile Tyr Leu Ser Thr Leu Trp Tyr Lys Pro
   65                  70                  75                  80
   Pro Phe Leu Ser Asp Phe Asn Asn Gly Ile Phe Ser Lys Val Lys Asn
                   85                  90                  95
   Thr Lys Leu Tyr Val Asn Asn Thr Leu Tyr Ser Glu Phe Ser Thr Ile
               100                 105                 110
   Val Ile Gly Ser Val Phe Val Asn Thr Ser Tyr Thr Ile Val Val Gln
           115                 120                 125
   Pro His Asn Gly Ile Leu Glu Ile Thr Ala Cys Gln Tyr Thr Met Cys
       130                 135                 140
   Glu Tyr Pro His Thr Val Cys Lys Ser Lys Gly Ser Ile Arg Asn Glu
   145                 150                 155                 160
   Ser Trp His Ile Asp Ser Ser Glu Pro Leu Cys Leu Phe Lys Lys Asn
                   165                 170                 175
   Phe Thr Tyr Asn Val Ser Ala Asp Trp Leu Tyr Phe His Phe Tyr Gln
               180                 185                 190
   Glu Arg Gly Val Phe Tyr Ala Tyr Tyr Ala Asp Val Gly Met Pro Thr
           195                 200                 205
   Thr Phe Leu Phe Ser Leu Tyr Leu Gly Thr Ile Leu Ser His Tyr Tyr
       210                 215                 220
   Val Met Pro Leu Thr Cys Asn Ala Ile Ser Ser Asn Thr Asp Asn Glu
   225                 230                 235                 240
   Thr Leu Glu Tyr Trp Val Thr Pro Leu Ser Arg Arg Gln Tyr Leu Leu
                   245                 250                 255
   Asn Phe Asp Glu His Gly Val Ile Thr Asn Ala Val Asp Cys Ser Ser
               260                 265                 270
   Ser Phe Leu Ser Glu Ile Gln Cys Lys Thr Gln Ser Phe Ala Pro Asn
           275                 280                 285
   Thr Gly Val Tyr Asp Leu Ser Gly Phe Thr Val Lys Pro Val Ala Thr
       290                 295                 300
   Val Tyr Arg Arg Ile Pro Asn Leu Pro Asp Cys Asp Ile Asp Asn Trp
   305                 310                 315                 320
   Leu Asn Asn Val Ser Val Pro Ser Pro Leu Asn Trp Glu Arg Arg Ile
                   325                 330                 335
   Phe Ser Asn Cys Asn Phe Asn Leu Ser Thr Leu Leu Arg Leu Val His
               340                 345                 350
   Val Asp Ser Phe Ser Cys Asn Asn Leu Asp Lys Ser Lys Ile Phe Gly
           355                 360                 365
   Ser Cys Phe Asn Ser Ile Thr Val Asp Lys Phe Ala Ile Pro Asn Arg
       370                 375                 380
   Arg Arg Asp Asp Leu Gln Leu Gly Ser Ser Gly Phe Leu Gln Ser Ser
   385                 390                 395                 400
   Asn Tyr Lys Ile Asp Ile Ser Ser Ser Ser Cys Gln Leu Tyr Tyr Ser
                   405                 410                 415
   Leu Pro Leu Val Asn Val Thr Ile Asn Asn Phe Asn Pro Ser Ser Trp
               420                 425                 430
   Asn Arg Arg Tyr Gly Phe Gly Ser Phe Asn Leu Ser Ser Tyr Asp Val
           435                 440                 445
   Val Tyr Ser Asp His Cys Phe Ser Val Asn Ser Asp Phe Cys Pro Cys
       450                 455                 460
   Ala Asp Pro Ser Val Val Asn Ser Cys Ala Lys Ser Lys Pro Pro Ser
   465                 470                 475                 480
   Ala Ile Cys Pro Ala Gly Thr Lys Tyr Arg His Cys Asp Leu Asp Thr
                   485                 490                 495
   Thr Leu Tyr Val Lys Asn Trp Cys Arg Cys Ser Cys Leu Pro Asp Pro
               500                 505                 510
   Ile Ser Thr Tyr Ser Pro Asn Thr Cys Pro Gln Lys Lys Val Val Val
           515                 520                 525
   Gly Ile Gly Glu His Cys Pro Gly Leu Gly Ile Asn Glu Glu Lys Cys
       530                 535                 540
   Gly Thr Gln Leu Asn His Ser Ser Cys Phe Cys Ser Pro Asp Ala Phe
   545                 550                 555                 560
   Leu Gly Trp Ser Phe Asp Ser Cys Ile Ser Asn Asn Arg Cys Asn Ile
                   565                 570                 575
   Phe Ser Asn Phe Ile Phe Asn Gly Ile Asn Ser Gly Thr Thr Cys Ser
               580                 585                 590
   Asn Asp Leu Leu Tyr Ser Asn Thr Glu Ile Ser Thr Gly Val Cys Val
           595                 600                 605
   Asn Tyr Asp Leu Tyr Gly Ile Thr Gly Gln Gly Ile Phe Lys Glu Val
       610                 615                 620
   Ser Ala Ala Tyr Tyr Asn Asn Trp Gln Asn Leu Leu Tyr Asp Ser Asn
   625                 630                 635                 640
   Gly Asn Ile Ile Gly Phe Lys Asp Phe Leu Thr Asn Lys Thr Tyr Thr
                   645                 650                 655
   Ile Leu Pro Cys Tyr Ser Gly Arg Val Ser Ala Ala Phe Tyr Gln Asn
               660                 665                 670
   Ser Ser Ser Pro Ala Leu Leu Tyr Arg Asn Leu Lys Cys Ser Tyr Val
           675                 680                 685
   Leu Asn Asn Ile Ser Phe Ile Ser Gln Pro Phe Tyr Phe Asp Ser Tyr
       690                 695                 700
   Leu Gly Cys Val Leu Asn Ala Val Asn Leu Thr Ser Tyr Ser Val Ser
   705                 710                 715                 720
   Ser Cys Asp Leu Arg Met Gly Ser Gly Phe Cys Ile Asp Tyr Ala Leu
                   725                 730                 735
   Pro Ser Ser Arg Arg Lys Arg Arg
               740
<210> 10
   <211> 702
   <212> PRT
   <213> Artificial Sequence
<220>
   <223> S1 [HCoV-NL63]
<400> 10
   Phe Phe Thr Cys Asn Ser Asn Ala Asn Leu Ser Met Leu Gln Leu Gly
   1               5                   10                  15
   Val Pro Asp Asn Ser Ser Thr Ile Val Thr Gly Leu Leu Pro Thr His
               20                  25                  30
   Trp Phe Cys Ala Asn Gln Ser Thr Ser Val Tyr Ser Ala Asn Gly Phe
           35                  40                  45
   Phe Tyr Ile Asp Val Gly Asn His Arg Ser Ala Phe Ala Leu His Thr
       50                  55                  60
   Gly Tyr Tyr Asp Ala Asn Gln Tyr Tyr Ile Tyr Val Thr Asn Glu Ile
   65                  70                  75                  80
   Gly Leu Asn Ala Ser Val Thr Leu Lys Ile Cys Lys Phe Ser Arg Asn
                   85                  90                  95
   Thr Thr Phe Asp Phe Leu Ser Asn Ala Ser Ser Ser Phe Asp Cys Ile
               100                 105                 110
   Val Asn Leu Leu Phe Thr Glu Gln Leu Gly Ala Pro Leu Gly Ile Thr
           115                 120                 125
   Ile Ser Gly Glu Thr Val Arg Leu His Leu Tyr Asn Val Thr Arg Thr
       130                 135                 140
   Phe Tyr Val Pro Ala Ala Tyr Lys Leu Thr Lys Leu Ser Val Lys Cys
   145                 150                 155                 160
   Tyr Phe Asn Tyr Ser Cys Val Phe Ser Val Val Asn Ala Thr Val Thr
                   165                 170                 175
   Val Asn Val Thr Thr His Asn Gly Arg Val Val Asn Tyr Thr Val Cys
               180                 185                 190
   Asp Asp Cys Asn Gly Tyr Thr Asp Asn Ile Phe Ser Val Gln Gln Asp
           195                 200                 205
   Gly Arg Ile Pro Asn Gly Phe Pro Phe Asn Asn Trp Phe Leu Leu Thr
       210                 215                 220
   Asn Gly Ser Thr Leu Val Asp Gly Val Ser Arg Leu Tyr Gln Pro Leu
   225                 230                 235                 240
   Arg Leu Thr Cys Leu Trp Pro Val Pro Gly Leu Lys Ser Ser Thr Gly
                   245                 250                 255
   Phe Val Tyr Phe Asn Ala Thr Gly Ser Asp Val Asn Cys Asn Gly Tyr
               260                 265                 270
   Gln His Asn Ser Val Val Asp Val Met Arg Tyr Asn Leu Asn Phe Ser
           275                 280                 285
   Ala Asn Ser Leu Asp Asn Leu Lys Ser Gly Val Ile Val Phe Lys Thr
       290                 295                 300
   Leu Gln Tyr Asp Val Leu Phe Tyr Cys Ser Asn Ser Ser Ser Gly Val
   305                 310                 315                 320
   Leu Asp Thr Thr Ile Pro Phe Gly Pro Ser Ser Gln Pro Tyr Tyr Cys
                   325                 330                 335
   Phe Ile Asn Ser Thr Ile Asn Thr Thr His Val Ser Thr Phe Val Gly
               340                 345                 350
   Ile Leu Pro Pro Thr Val Arg Glu Ile Val Val Ala Arg Thr Gly Gln
           355                 360                 365
   Phe Tyr Ile Asn Gly Phe Lys Tyr Phe Asp Leu Gly Phe Ile Glu Ala
       370                 375                 380
   Val Asn Phe Asn Val Thr Thr Ala Ser Ala Thr Asp Phe Trp Thr Val
   385                 390                 395                 400
   Ala Phe Ala Thr Phe Val Asp Val Leu Val Asn Val Ser Ala Thr Asn
                   405                 410                 415
   Ile Gln Asn Leu Leu Tyr Cys Asp Ser Pro Phe Glu Lys Leu Gln Cys
               420                 425                 430
   Glu His Leu Gln Phe Gly Leu Gln Asp Gly Phe Tyr Ser Ala Asn Phe
           435                 440                 445
   Leu Asp Asp Asn Val Leu Pro Glu Thr Tyr Val Ala Leu Pro Ile Tyr
       450                 455                 460
   Tyr Gln His Thr Asp Ile Asn Phe Thr Ala Thr Ala Ser Phe Gly Gly
   465 470 475 480
   Ser Cys Tyr Val Cys Lys Pro His Gln Val Asn Ile Ser Leu Asn Gly
   485 490 495
   Asn Thr Ser Val Cys Val Arg Thr Ser His Phe Ser Ile Arg Tyr Ile
   500 505 510
   Tyr Asn Arg Val Lys Ser Gly Ser Pro Gly Asp Ser Ser Trp His Ile
   515 520 525
   Tyr Leu Lys Ser Gly Thr Cys Pro Phe Ser Phe Ser Lys Leu Asn Asn
   530 535 540
   Phe Gln Lys Phe Lys Thr Ile Cys Phe Ser Thr Val Glu Val Pro Gly
   545 550 555 560
   Ser Cys Asn Phe Pro Leu Glu Ala Thr Trp His Tyr Thr Ser Tyr Thr
   565 570 575
   Ile Val Gly Ala Leu Tyr Val Thr Trp Ser Glu Gly Asn Ser Ile Thr
   580 585 590
   Gly Val Pro Tyr Pro Val Ser Gly Ile Arg Glu Phe Ser Asn Leu Val
   595 600 605
   Leu Asn Asn Cys Thr Lys Tyr Asn Ile Tyr Asp Tyr Val Gly Thr Gly
   610 615 620
   Ile Ile Arg Ser Ser Asn Gln Ser Leu Ala Gly Gly Ile Thr Tyr Val
   625 630 635 640
   Ser Asn Ser Gly Asn Leu Leu Gly Phe Lys Asn Val Ser Thr Gly Asn
   645 650 655
   Ile Phe Ile Val Thr Pro Cys Asn Gln Pro Asp Gln Val Ala Val Tyr
   660 665 670
   Gln Gln Ser Ile Ile Gly Ala Met Thr Ala Val Asn Glu Ser Arg Tyr
   675 680 685
   Gly Leu Gln Asn Leu Leu Gln Leu Pro Asn Phe Tyr Tyr Val
   690 695 700
<210> 11
   <211> 657
   <212> PRT
   <213> Artificial Sequence
<220>
   <223> S1 [SARS_CoV]
<400> 11
   Ser Gly Ser Asp Leu Asp Arg Cys Thr Thr Phe Asp Asp Val Gln Ala
   1               5                   10                  15
   Pro Asn Tyr Thr Gln His Thr Ser Ser Met Arg Gly Val Tyr Tyr Pro
               20                  25                  30
   Asp Glu Ile Phe Arg Ser Asp Thr Leu Tyr Leu Thr Gln Asp Leu Phe
           35                  40                  45
   Leu Pro Phe Tyr Ser Asn Val Thr Gly Phe His Thr Ile Asn His Thr
       50                  55                  60
   Phe Gly Asn Pro Val Ile Pro Phe Lys Asp Gly Ile Tyr Phe Ala Ala
   65                  70                  75                  80
   Thr Glu Lys Ser Asn Val Val Arg Gly Trp Val Phe Gly Ser Thr Met
                   85                  90                  95
   Asn Asn Lys Ser Gln Ser Val Ile Ile Ile Asn Asn Ser Thr Asn Val
               100                 105                 110
   Val Ile Arg Ala Cys Asn Phe Glu Leu Cys Asp Asn Pro Phe Phe Ala
           115                 120                 125
   Val Ser Lys Pro Met Gly Thr Gln Thr His Thr Met Ile Phe Asp Asn
       130                 135                 140
   Ala Phe Asn Cys Thr Phe Glu Tyr Ile Ser Asp Ala Phe Ser Leu Asp
   145                 150                 155                 160
   Val Ser Glu Lys Ser Gly Asn Phe Lys His Leu Arg Glu Phe Val Phe
                   165                 170                 175
   Lys Asn Lys Asp Gly Phe Leu Tyr Val Tyr Lys Gly Tyr Gln Pro Ile
               180                 185                 190
   Asp Val Val Arg Asp Leu Pro Ser Gly Phe Asn Thr Leu Lys Pro Ile
           195                 200                 205
   Phe Lys Leu Pro Leu Gly Ile Asn Ile Thr Asn Phe Arg Ala Ile Leu
       210                 215                 220
   Thr Ala Phe Ser Pro Ala Gln Asp Ile Trp Gly Thr Ser Ala Ala Ala
   225 230 235 240
   Tyr Phe Val Gly Tyr Leu Lys Pro Thr Thr Phe Met Leu Lys Tyr Asp
   245 250 255
   Glu Asn Gly Thr Ile Thr Asp Ala Val Asp Cys Ser Gln Asn Pro Leu
   260 265 270
   Ala Glu Leu Lys Cys Ser Val Lys Ser Phe Glu Ile Asp Lys Gly Ile
   275 280 285
   Tyr Gln Thr Ser Asn Phe Arg Val Val Pro Ser Gly Asp Val Val Arg
   290 295 300
   Phe Pro Asn Ile Thr Asn Leu Cys Pro Phe Gly Glu Val Phe Asn Ala
   305 310 315 320
   Thr Lys Phe Pro Ser Val Tyr Ala Trp Glu Arg Lys Lys Ile Ser Asn
   325 330 335
   Cys Val Ala Asp Tyr Ser Val Leu Tyr Asn Ser Thr Phe Phe Ser Thr
   340 345 350
   Phe Lys Cys Tyr Gly Val Ser Ala Thr Lys Leu Asn Asp Leu Cys Phe
   355 360 365
   Ser Asn Val Tyr Ala Asp Ser Phe Val Val Lys Gly Asp Asp Val Arg
   370 375 380
   Gln Ile Ala Pro Gly Gln Thr Gly Val Ile Ala Asp Tyr Asn Tyr Lys
   385 390 395 400
   Leu Pro Asp Asp Phe Met Gly Cys Val Leu Ala Trp Asn Thr Arg Asn
   405 410 415
   Ile Asp Ala Thr Ser Thr Gly Asn Tyr Asn Tyr Lys Tyr Arg Tyr Leu
   420 425 430
   Arg His Gly Lys Leu Arg Pro Phe Glu Arg Asp Ile Ser Asn Val Pro
   435 440 445
   Phe Ser Pro Asp Gly Lys Pro Cys Thr Pro Pro Ala Leu Asn Cys Tyr
   450 455 460
   Trp Pro Leu Asn Asp Tyr Gly Phe Tyr Thr Thr Thr Gly Ile Gly Tyr
   465 470 475 480
   Gln Pro Tyr Arg Val Val Val Leu Ser Phe Glu Leu Leu Asn Ala Pro
   485 490 495
   Ala Thr Val Cys Gly Pro Lys Leu Ser Thr Asp Leu Ile Lys Asn Gln
   500 505 510
   Cys Val Asn Phe Asn Phe Asn Gly Leu Thr Gly Thr Gly Val Leu Thr
   515 520 525
   Pro Ser Ser Lys Arg Phe Gln Pro Phe Gln Gln Phe Gly Arg Asp Val
   530 535 540
   Ser Asp Phe Thr Asp Ser Val Arg Asp Pro Lys Thr Ser Glu Ile Leu
   545 550 555 560
   Asp Ile Ser Pro Cys Ser Phe Gly Gly Val Ser Val Ile Thr Pro Gly
   565 570 575
   Thr Asn Ala Ser Ser Glu Val Ala Val Leu Tyr Gln Asp Val Asn Cys
   580 585 590
   Thr Asp Val Ser Thr Ala Ile His Ala Asp Gln Leu Thr Pro Ala Trp
   595 600 605
   Arg Ile Tyr Ser Thr Gly Asn Asn Val Phe Gln Thr Gln Ala Gly Cys
   610 615 620
   Leu Ile Gly Ala Glu His Val Asp Thr Ser Tyr Glu Cys Asp Ile Pro
   625 630 635 640
   Ile Gly Ala Gly Ile Cys Ala Ser Tyr His Thr Val Ser Leu Leu Arg
   645 650 655
   Leu
<210> 12
   <211> 649
   <212> PRT
   <213> Artificial Sequence
<220>
   <223> fragment of SEQ ID NO1
<400> 12
   Asn Ser Phe Thr Arg Gly Val Tyr Tyr Pro Asp Lys Val Phe Arg Ser
   1               5                   10                  15
   Ser Val Leu His Ser Thr Gln Asp Leu Phe Leu Pro Phe Phe Ser Asn
   20 25 30
   Val Thr Trp Phe His Ala Ile His Val Ser Gly Thr Asn Gly Thr Lys
   35 40 45
   Arg Phe Asp Asn Pro Val Leu Pro Phe Asn Asp Gly Val Tyr Phe Ala
   50 55 60
   Ser Thr Glu Lys Ser Asn Ile Ile Arg Gly Trp Ile Phe Gly Thr Thr
   65 70 75 80
   Leu Asp Ser Lys Thr Gln Ser Leu Leu Ile Val Asn Asn Ala Thr Asn
   85 90 95
   Val Val Ile Lys Val Cys Glu Phe Gln Phe Cys Asn Asp Pro Phe Leu
   100 105 110
   Gly Val Tyr Tyr His Lys Asn Asn Lys Ser Trp Met Glu Ser Glu Phe
   115 120 125
   Arg Val Tyr Ser Ser Ala Asn Asn Cys Thr Phe Glu Tyr Val Ser Gln
   130 135 140
   Pro Phe Leu Met Asp Leu Glu Gly Lys Gln Gly Asn Phe Lys Asn Leu
   145 150 155 160
   Arg Glu Phe Val Phe Lys Asn Ile Asp Gly Tyr Phe Lys Ile Tyr Ser
   165 170 175
   Lys His Thr Pro Ile Asn Leu Val Arg Asp Leu Pro Gln Gly Phe Ser
   180 185 190
   Ala Leu Glu Pro Leu Val Asp Leu Pro Ile Gly Ile Asn Ile Thr Arg
   195 200 205
   Phe Gln Thr Leu Leu Ala Leu His Arg Ser Tyr Leu Thr Pro Gly Asp
   210 215 220
   Ser Ser Ser Gly Trp Thr Ala Gly Ala Ala Ala Tyr Tyr Val Gly Tyr
   225 230 235 240
   Leu Gln Pro Arg Thr Phe Leu Leu Lys Tyr Asn Glu Asn Gly Thr Ile
   245 250 255
   Thr Asp Ala Val Asp Cys Ala Leu Asp Pro Leu Ser Glu Thr Lys Cys
   260 265 270
   Thr Leu Lys Ser Phe Thr Val Glu Lys Gly Ile Tyr Gln Thr Ser Asn
   275 280 285
   Phe Arg Val Gln Pro Thr Glu Ser Ile Val Arg Phe Pro Asn Ile Thr
   290 295 300
   Asn Leu Cys Pro Phe Gly Glu Val Phe Asn Ala Thr Arg Phe Ala Ser
   305 310 315 320
   Val Tyr Ala Trp Asn Arg Lys Arg Ile Ser Asn Cys Val Ala Asp Tyr
   325 330 335
   Ser Val Leu Tyr Asn Ser Ala Ser Phe Ser Thr Phe Lys Cys Tyr Gly
   340 345 350
   Val Ser Pro Thr Lys Leu Asn Asp Leu Cys Phe Thr Asn Val Tyr Ala
   355 360 365
   Asp Ser Phe Val Ile Arg Gly Asp Glu Val Arg Gln Ile Ala Pro Gly
   370 375 380
   Gln Thr Gly Lys Ile Ala Asp Tyr Asn Tyr Lys Leu Pro Asp Asp Phe
   385 390 395 400
   Thr Gly Cys Val Ile Ala Trp Asn Ser Asn Asn Leu Asp Ser Lys Val
   405 410 415
   Gly Gly Asn Tyr Asn Tyr Leu Tyr Arg Leu Phe Arg Lys Ser Asn Leu
   420 425 430
   Lys Pro Phe Glu Arg Asp Ile Ser Thr Glu Ile Tyr Gln Ala Gly Ser
   435 440 445
   Thr Pro Cys Asn Gly Val Glu Gly Phe Asn Cys Tyr Phe Pro Leu Gln
   450 455 460
   Ser Tyr Gly Phe Gln Pro Thr Asn Gly Val Gly Tyr Gln Pro Tyr Arg
   465 470 475 480
   Val Val Val Leu Ser Phe Glu Leu Leu His Ala Pro Ala Thr Val Cys
   485 490 495
   Gly Pro Lys Lys Ser Thr Asn Leu Val Lys Asn Lys Cys Val Asn Phe
   500 505 510
   Asn Phe Asn Gly Leu Thr Gly Thr Gly Val Leu Thr Glu Ser Asn Lys
   515 520 525
   Lys Phe Leu Pro Phe Gln Gln Phe Gly Arg Asp Ile Ala Asp Thr Thr
       530                 535                 540
   Asp Ala Val Arg Asp Pro Gln Thr Leu Glu Ile Leu Asp Ile Thr Pro
   545                 550                 555                 560
   Cys Ser Phe Gly Gly Val Ser Val Ile Thr Pro Gly Thr Asn Thr Ser
                   565                 570                 575
   Asn Gln Val Ala Val Leu Tyr Gln Asp Val Asn Cys Thr Glu Val Pro
               580                 585                 590
   Val Ala Ile His Ala Asp Gln Leu Thr Pro Thr Trp Arg Val Tyr Ser
           595                 600                 605
   Thr Gly Ser Asn Val Phe Gln Thr Arg Ala Gly Cys Leu Ile Gly Ala
       610                 615                 620
   Glu His Val Asn Asn Ser Tyr Glu Cys Asp Ile Pro Ile Gly Ala Gly
   625                 630                 635                 640
   Ile Cys Ala Ser Tyr Gln Thr Gln Thr
                   645